# EUROPÄISCHE PATENTSCHRIFT

(11) **EP 2 461 860 B1**
(45) Veröffentlichungstag und Bekanntmachung des Hinweises auf die Patenterteilung: **16.01.2019**
(21) Anmeldenummer: 10742724.7
(22) Anmeldetag: 23.07.2010
(51) Int. Cl.: A61M 15/08

(54) **AUSTRAGVORRICHTUNG FÜR FLÜSSIGE MEDIEN**
DISCHARGE DEVICE FOR LIQUID MEDIA
DISPOSITIF DE DISTRIBUTION D'AGENTS LIQUIDES

(30) Priorität: 03.08.2009 DE 102009037164
(43) Veröffentlichungstag der Anmeldung: 13.06.2012
(73) Patentinhaber: Aptar Radolfzell GmbH, 78315 Radolfzell (DE)
(72) Erfinder: GREINER-PERTH, Jürgen, 78244 Gottmadingen (DE); BIEG, Jörg, 78112 St. Georgen (DE)
(74) Vertreter: Patentanwaltskanzlei Cartagena
(86) Internationale Anmeldenummer: PCT/EP2010/004516
(87) Internationale Veröffentlichungsnummer: WO 2011/015292

(56) Entgegenhaltungen:
- EP-A1- 0 818 247
- EP-A2- 1 616 631
- WO-A1-03/078073
- WO-A2-2007/096049
- WO-A2-2007/138084
- DE-A1- 2 604 001
- DE-A1- 10 023 551
- DE-A1-102005 033 771
- FR-A1- 2 736 285
- FR-A1- 2 738 553
- US-A- 3 949 939
- US-B1- 6 269 976

## Beschreibung

### ANWENDUNGSGEBIET UND STAND DER TECHNIK

Die Erfindung betrifft ein Set zur Bildung einer Austrageinheit zum Austrag pharmazeutischer Medien aus einem Medienspeicher mit einer ersten Austragöffnung, einer Pumpe, einem Einlasskanal zur Verbindung des Medienspeichers mit der Pumpe und einem Auslasskanal zur Verbindung der Pumpe mit der ersten Austragöffnung.

Derartige Austrageinheiten sind aus dem Stand der Technik allgemein bekannt. Sie werden üblicherweise mit einem Medienspeicher verbunden, der entweder im Gehäuse der Austrageinheit aufgenommen wird, oder auf den die Austrageinheit aufgesetzt wird. Anschließend kann mittels der Pumpe Medium aus dem Medienspeicher bis zur Austrittsöffnung gefördert werden, von wo es üblicherweise in zerstäubter Form an die Umgebung abgegeben wird. Derartige Austrageinheiten können insbesondere als nasale Austrageinheiten ausgebildet sein. Als solche weisen sie eine zum Einführen in ein Nasenloch eines Patienten vorgesehene Nasenolive auf, an der die erste Austrittsöffnung vorgesehen ist.

Sofern eine Austrageinheit nur von einem Patienten benutzt wird, müssen naturgemäß keine Maßnahmen getroffen werden, um eine Kontamination des Patienten durch einen anderen Patienten, der die gleiche Austrageinheit verwendet, zu verhindern. Problematisch ist die Verwendung einer solchen Austrageinheit für mehrere Patienten, beispielsweise im Rahmen einer Verwendung mit einem Impfstoff, da dies mit der Gefahr einhergeht, dass Krankheitserreger des einen Patienten nach dessen Benutzung an der Austrageinheit verbleiben und bei einem die Austrageinheit nachfolgend verwendenden Patienten zu einer Infektion führen.

Zwar ist denkbar, dass bei einer üblichen Austrageinheit der Austragkopf mit der Nasenolive ausgetauscht wird, bevor die Austrageinheit durch einen weiteren Patienten verwendet wird. Da jedoch der Austragkopf bekannter Austrageinheiten einen erheblichen Teil des Austragkanals aufweisen, führt dies gleichzeitig auch zu einem nicht unerheblichen Verlust von pharmazeutischem Medium. Hinzu kommt, dass nach einem solchen Austausch des Austragkopfes zunächst der Auslasskanal wieder vollständig mit Medium gefüllt sein muss, um nachfolgend eine wunschgemäße Dosierung zu erzielen. Daher würde es nach dem Austausch des Austragkopfes bekannter Austrageinheiten zunächst wieder des sogenannten Primens bedürfen, also der Durchführung einiger Pumpenhübe, um vor der Inbetriebnahme den gesamten Austragkanal mit Medium zu füllen. Auch hierbei würde ein nicht unerheblicher Anteil des pharmazeutischen Mediums verloren gehen.

Während der Verlust des pharmazeutischen Mediums bei vielerlei Anwendungszwecken lediglich bedauerlich ist, ist es im Zusammenhang mit Massenimpfungen bei Pandemien aufgrund des häufig nur beschränkt verfügbaren Wirkstoffes nicht akzeptabel, dass in oben genannter Weise ein erheblicher Teil des Wirkstoffs verloren geht.

Ein sich ebenfalls im Kontext von Massenimpfungen einstellendes Problem liegt darin, dass bei Massenimpfungen sehr kurzfristig große Stückzahlen an Austrageinheiten zum Austragen des pharmazeutischen Mediums zur Verfügung gestellt werden müssen. Dabei ist möglicherweise nicht immer die Zeit gegeben, um diese große Stückzahlen an Austrageinheiten herzustellen. Die präventive Lagerhaltung einer großen Zahl von Austrageinheiten stellt allerdings keine befriedigend Lösung dar, da der an bekannter Austrageinheiten üblicherweise vorgesehene Verbindungsanschluss zur Verbindung mit einem Medienspeicher an den jeweiligen Typ des Mediumspeichers angepasst sein muss. Die sich daraus ergebenden fehlende Flexibilität hinsichtlich der Verwendung einer Austrageinheit wird ebenfalls als nachteilig angesehen.

Aus der WO 03/078073 A1 ist ein Spender bekannt, der über einen aufgesetzten Austragkopf verfügt, der beim Ziehen isoliert entfernbar ist, so dass das Risiko einer Kontamination von Flüssigkeit begrenzt ist.

Aus der DE 10 2005 033 771 A1 ist ein Spender bekannt, der einen Austragstutzen mit Austragöffnung aufweist, der mittels einer Schnappverbindung an einer Innenhülse befestigt ist. Eine Wirbelkammer ist zwischen einem auf Seiten der Innenhülse vorgesehenen Ventilstopfens und der Austragstutzen vorgesehen.

Aus der FR 2 736 285 A1 geht ebenfalls ein Spender hervor, bei der ein Austragstutzen an einer Innenhülse befestigt ist. Der Austragstutzen umfasst dabei einen Zerstäuber. Aus der DE 26 04 001 A1 ist ebenfalls Spender mit einem Zerstäuber bekannt.

### AUFGABE UND LÖSUNG

Aufgabe der Erfindung ist es daher, eine beschriebene Austrageinheit dahingehend weiterzubilden bzw. zu ergänzen, dass sie für die Verwendung von mehreren Patienten geeignet ist.

Hinsichtlich der Verwendbarkeit einer Austrageinheit durch mehrere Patienten bei gleichzeitiger Vermeidung von sogenannten Querkontaminationen liegt der Grundgedanke der vorliegenden Erfindung darin, dass der Teil der Austrageinheit, der jeweils immer nur für einen Patienten verwendet wird, so zu gestalten ist, dass mit dem Austausch nur ein kleiner Teil des pharmazeutischen Medium verloren geht. Um ohne zwischenzeitliches Primen die Austrageinheit unmittelbar nacheinander für zwei Patienten verwenden zu können, ist es vorgesehen, dass maximal 15 µl des Mediums mit dem Austausch der auszutauschenden Teile verloren geht.

Vorgeschlagen wird ein Set aus mindestens zwei ersten Teileinrichtungen und einer zweiten Teileinrichtung zur Bildung einer Austrageinheit.

Bei einem ersten Konzept der Erfindung ist die erste Austrittsöffnung Teil jeweils der Teileinrichtungen der Austrageinheit. Die Pumpe, zumindest ein Teilabschnitt des Auslasskanals sowie ein im Auslasskanal vorgesehenes Auslassventil sind Teil der zweiten Teileinrichtung der Austrageinheit. Dabei sind die ersten Teileinrichtungen als werkzeuglos an die zweite Teileinrichtung ankoppelbare und ebenfalls werkzeuglos von der zweiten Teileinrichtung entkoppelbare Teileinrichtungen ausgebildet. Weiterhin ist ein Innenvolumen eines Teilabschnitts des Auslasskanals, der sich von dem Auslassventil zur ersten Austrittsöffnung erstreckt, kleiner als 15 µl, vorzugsweise kleiner als 5 µl, insbesondere vorzugsweise kleiner als 1 µl.

Bei diesem ersten Konzept ist demnach vorgesehen, dass die zweite Teileinrichtung, die zur Wiederverwendung vorgesehen ist und die daher die Pumpe enthält, ein vorzugsweise mediendruckabhängig öffnendes Auslassventil aufweist, welches so nahe an einer Schnittstelle zur zweiten Teileinrichtung angeordnet ist, dass das gegebenenfalls kontaminierte Medium in einem Auslasskanalabschnitt der ersten Teileinrichtungen und/oder der zweiten Teileinrichtung jenseits des Auslassventils maximal 15 µl beträgt. Als Auslasskanal im Sinne dieser Erfindung wird dabei der Teil des Flüssigkeitswegs von der Pumpe bis zur ersten Austrittsöffnung angesehen, der während der Verwendung der Austrageinheit mit einem nur aus dem flüssigen Medium bestehenden Volumenstrom gefüllt ist. Das Ende des Auslasskanals kann beispielsweise durch den Teil einer Verwirbelungseinrichtung definiert sind, ab dem die Flüssigkeit nicht mehr als reiner Flüssigkeitsvolumenstroms vorliegt, sondern insbesondere in zerstäubter Form aus kleinen Tröpfchen besteht.

Bei diesem ersten Konzept der Erfindung liegt die Besonderheit somit darin, dass das Auslassventil, welches nach einem Austragvorgang den potentiell kontaminierten Teil des Mediums von dem in jedem Fall unkontaminierten Teil des Mediums trennt, unmittelbar vor der Schnittstelle der zweiten zur ersten Teileinrichtung angeordnet ist. Dabei sind sowohl Gestaltungen denkbar, bei denen der Auslasskanal gemäß oben genannter Definition noch im Bereich der zweiten Teileinrichtung endet, als auch solche Gestaltungen, bei der Auslasskanal sich bis in die erste Teileinrichtung erstreckt. Bei den erstgenannten Gestaltungen ist die Austragöffnung an der ersten Teileinrichtung bereits jenseits einer Verwirbelungseinrichtung an der zweiten Teileinrichtung vorgesehen und wird beim bestimmungsgemäßen Gebrauch bereits durch Medium in versprühter Form durchquert.

Bei einem zweiten Konzept der Erfindung ist das Set dadurch weitergebildet, dass die erste Austragöffnung, zumindest ein Teilabschnitt des Auslasskanals sowie eine im Auslasskanal vorgesehene erste Verwirbelungseinrichtung Teil jeweils der ersten Teileinrichtungen sind und wobei die Pumpe und zumindest ein Teilabschnitt des Auslasskanals Teil der zweiten Teileinrichtung sind. Dabei ist die erste Teileinrichtung als werkzeuglos an die zweite Teileinrichtung ankoppelbare und von der zweiten Teileinrichtung ebenfalls werkzeuglos entkoppelbare Teileinrichtung ausgebildet. Weiterhin ist ein Innenvolumen des in der ersten Teileinrichtung vorgesehenen Teilabschnitts des Auslasskanals kleiner als 15 µl, vorzugsweise kleiner als 5 µl, insbesondere vorzugsweise kleiner als 1 µl.

Besonderheit bei diesem zweiten Konzept ist somit, dass die nicht zur Wiederverwendung vorgesehenen ersten Teileinrichtungen die erste Verwirbelungseinrichtung aufweist, ab der das Medium nicht mehr als einheitlicher Flüssigkeitsvolumenstrom weitergefördert wird. Es wurde festgestellt, dass die Verwirbelungseinrichtung einen sehr guten Schutz gegen Kontamination darstellt. Selbst wenn durch einen infizierten Patienten eine Kontamination der Verwirbelungseinrichtung eintritt, so setzt sich diese üblicherweise nicht bis in den zur ersten Teileinrichtung gehörenden Teilabschnitt des Auslasskanals vor der Verwirbelungseinrichtung fort. Dieser Teilabschnitt des Auslasskanals in den ersten Teileinrichtungen und das darin nach einem Austragvorgang noch befindliche Medium stellen eine wirksame Barriere dagegen dar, dass die Kontamination sich bis zur zweiten Teileinrichtung der Austrageinheit fortpflanzt. Es kann daher durchaus zweckmäßig sein, das Innenvolumen des in der ersten Teileinrichtung vorgesehenen Abschnitts des Auslasskanals zwar gering zu halten, es jedoch auf etwa 3 µl bis 5 µl zu bemessen, so dass die genannte Barrierefunktion zuverlässig erfüllt wird.

Um ein höheres Maß an Sicherheit zu erreichen, kann zusätzlich vorgesehen sein, dass die ersten Teileinrichtungen ein vorzugsweise mediendruckabhängig öffnendes Ventil aufweist, welches auch dann eine Kontamination der zweiten Teileinrichtung verhindert, wenn die Teileinrichtungen nach einem mit einer Kontamination der ersten Teileinrichtung einhergehenden Austragvorgang noch für längere Zeit im gekoppelten Zustand verbleiben.

Als Verwirbelungseinrichtung im Sinne dieser Erfindung wird eine vom Auslasskanal mit Medium versorgte Einrichtung angesehen, die das Medium aus einem einheitlich flüssigen Volumenstrom in eine Austragform, insbesondere einen Sprühstrahl umformt. Insbesondere kann eine solche Verwirbelungseinrichtung schräggestellte Verwirbelungsflächen aufweisen, die der Flüssigkeit bezogen auf eine durch die mittlere Austragrichtung definierte Achse einen tangentialen Drall verleihen, der zur Ausbildung eines kegelförmigen Sprühstrahls führt.

Als im Sinne der genannten Konzepte werkzeuglos realisierbare Kopplung und Entkopplung wird es angesehen, wenn ohne den Einsatz von Hilfsmitteln, die über menschliche Hände hinausgehen, eine Trennung oder Fügung der Teileinrichtungen möglich ist. Dies kann durchaus auch mit einem zuvor erforderlichen Lösen von Verriegelungsmitteln wie Rastmitteln einhergehen, sofern dieses Lösen alleine durch Kraftbeaufschlagung von an der Austrageinheit zugänglichen Flächen möglich ist. Als besonders vorteilhaft wird es jedoch angesehen, wenn das Trennen und Fügen der Teileinrichtungen alleine durch die Kraftbeaufschlagung der Teileinrichtungen relativ zueinander in einer Trennrichtung bzw. einer Fügerichtung möglich ist, wobei die Kopplung vorzugsweise vollständig hinterschneidungsfrei ausgebildet ist.

Die werkzeuglose Handhabbarkeit der Teileinrichtungen ist erforderlich, um es dem Endanwender, beispielsweise dem Patienten oder einen Massenimpfungen verabreichenden Arzt, zu ermöglichen, die Austrageinheit kurzfristig für den nächsten Patienten vorzubereiten. Gerade bei Massenimpfungen, bei denen mitunter Tausende von Patienten geimpft werden müssen, ist eine umständliche Trennung der Teileinrichtungen mittels eines zusätzlichen Werkzeugs nicht akzeptabel. Als besonders vorteilhaft wird die Verwendung der genannten beiden Konzepte für Austrageinheiten mit Nasenapplikatoren angesehen, bei denen die erste Teileinrichtung einen Applikator, vorzugsweise in Form einer Nasenolive, aufweist, an dem die erste Austragöffnung vorgesehen ist.

Von besonderem Vorteil ist es, wenn auch die zweite Teileinrichtung, die bestimmungsgemäß wiederverwendet wird, einen Applikator, insbesondere in Form einer Nasenolive, mit einer zweiten Austragöffnung aufweist und die ersten Teileinrichtungen zum Aufsetzen auf diesen Applikator der zweiten Teileinrichtung ausgebildet ist. Bei einer solchen Gestaltung ist die zweite Teileinrichtung der Austrageinheit auch ohne eine erste Teileinrichtung verwendbar, da sie bereits selbst einen Applikator umfasst. Dieser Applikator wird bei Verwendung der Austrageinheit mit einer der ersten Teileinrichtungen zur Festlegung der jeweiligen ersten Teileinrichtung verwendet, indem die erste Teileinrichtung Berührflächen zur vorzugsweise kraftschlüssigen Festlegung an der zweiten Teileinrichtung umfasst, die an die Formgebung des Applikators der zweiten Teileinrichtung angepasstsind.

Weiterhin ist es von Vorteil, wenn die zweite Teileinrichtung eine der zweiten Austragöffnung vorgeschaltete zweite Verwirbelungseinrichtung aufweist. Diese zweite Verwirbelungseinrichtung erfüllt ihren bestimmungsgemäßen Zweck dann, wenn die zweite Teileinrichtung ohne eine erste Teileinrichtung als im Wesentlichen gattungsgemäße Austrageinheit verwendet wird. Wenn jedoch in der beschriebenen Art und Weise eine der ersten Teileinrichtungen an die zweite Teileinrichtung angekoppelt wird, verliert die zweite Verwirbelungseinrichtung ihren bestimmungsgemäßen Zweck und agiert lediglich als Teil des Auslasskanals.

Bei einer Weiterbildung der Erfindung, bei der die ersten Teileinrichtungen und die zweite Teileinrichtung jeweils eine Nasenolive aufweisen, verfügen diese Nasenoliven über voneinander abweichende Geometrien. Dabei wird als Geometrie die Außenform der Nasenolive angesehen, also die Formgebung aller Teile der Nasenolive, die bestimmungsgemäß mit der Nase des Patienten in Berührkontakt gelangen. Durch die Gestaltung mit verschiedenen Geometrien kann eine Austrageinheit eines erfindungsgemäßen Sets an verschiedene Patientengruppen, insbesondere an Erwachsene einerseits und Kinder andererseits, angepasst sein. In ähnlicher Weise wird es als vorteilhaft angesehen, wenn Austrageinheiten, bei denen die erste und die zweite Teileinrichtung jeweils eine Verwirbelungseinrichtung aufweisen, über unterschiedliche Verwirbelungseinrichtungen verfügen, die zur Erzeugung eines jeweils unterschiedlichen Sprühverhaltens ausgebildet sind. Dabei betrifft der Unterschied im Hinblick auf das Sprühverhalten insbesondere den Öffnungswinkel der Kegelform des Sprühstrahls und/oder die Tröpfchengröße, die durch die Verwirbelungseinrichtung erzeugt wird. Auch hier kann die Anpassung der Verwirbelungseinrichtungen insbesondere der Anpassung an die spezifischen Bedürfnisse von erwachsenen Patienten einerseits und Kindern andererseits dienen.

Als besonders vorteilhaft wird weiterhin angesehen, wenn die ersten Teileinrichtungen einen kappenförmigen ersten Außenkörper und einen darin eingesetzten Innenkörper aufweist. Hierdurch werden komplexere Strukturen innerhalb der ersten Teileinrichtungen fertigungstechnisch einfach möglich. Insbesondere kann der Innenkörper zumindest abschnittsweise die erste Verwirbelungseinrichtung bilden. Alternativ oder zusätzlich kann der Innenkörper eine Dichtfläche zur Verfügung stellen, die im gekoppelten Zustand der Teileinrichtungen an einer äußeren Oberfläche der zweiten Teileinrichtung anliegt. Insbesondere im Zusammenhang mit einer solchen Dichtfläche ist es von Vorteil, wenn der Innenkörper aus einem weicheren Material als der Außenkörper gefertigt ist.

Bei einer besonders bevorzugten Gestaltung der Erfindung sind an der ersten und an der zweiten Teileinrichtung jeweils eine Dichtfläche vorgesehen, die im gekoppelten Zustand der Teileinrichtungen aneinander anliegen und dadurch den Auslasskanal im Übergangsbereich zwischen den Teileinrichtungen nach außen umgebend gemeinsam abdichten. Dabei ist es besonders von Vorteil, wenn ein mittlerer Normalenvektor auf diesen Dichtflächen mit einer Entkopplungsrichtung der Teileinrichtungen einen Winkel kleiner 60° einschließt, vorzugsweise kleiner 30°, insbesondere kleiner 15°. Da die Teileinrichtungen vorzugsweise zum größten Teil aus Kunststoffteilen bestehen, die sich beim Koppeln der Teileinrichtungen leicht verformen, ist ein solcher vergleichsweise spitzer Winkel zwischen dem Normalenvektor und der Entkopplungsrichtung dienlich, um zu verhindern, dass sich beim Entkoppeln der Teileinrichtungen zwischen den Teileinrichtungen ein Unterdruck bildet, der bewirken könnte, dass kontaminiertes Medium aus der ersten Teileinrichtung angesogen wird und sich auf Außenflächen der zweiten Teileinrichtung niederschlägt.

Bei einer Austrageinheit eines erfindungsgemäßen Sets weist die zweite Teileinrichtung vorzugsweise zwei gegeneinander in einer Pumprichtung relativ bewegliche Baugruppen auf, die jeweils Bestandteile der Pumpe aufweisen, so dass ein Pumpvorgang durch Relativverlagerung dieser Baugruppen erzielbar ist.

Als besonders vorteilhaft wird es angesehen, wenn die erste Teileinrichtung, also der bestimmungsgemäß nicht wieder verwendbare Teil, eine Fingerauflage aufweist. Als eine solche Fingerauflage wird eine zumindest in etwa ebene Fläche angesehen, die eine Mindestgröße von 7 mm x 7 mm, insbesondere vorzugsweise eine größere Fläche, aufweist, wobei ein Normalenvektor auf dieser Fingerauflage mit der Pumprichtung einen Winkel von maximal 15° einschließt. Insbesondere kann die Fingerauflage als außen an dem kappenförmigen Außenbauteil der ersten Teileinrichtung angeformter oder angekoppelter Steg ausgebildet sein. Die Gestaltung der ersten Teileinrichtung mit einer solchen Fingerauflage bietet den Vorteil, dass während der Pumpbetätigung der Austrageinheit gleichzeitig auch die Teileinrichtungen aneinander gepresst werden, so dass eine zuverlässige Abdichtung zwischen den Teileinrichtungen gegeben ist. Trotz des Vorhandenseins einer solchen Fingerauflage an der ersten Teileinrichtung sind auch Gestaltungen zweckmäßig, die auch an der zweiten Teileinrichtung eine Fingerauflage aufweisen, wobei diese insbesondere der Verwendung der zweiten Teileinrichtung ohne die erste Teileinrichtung dient.

Erfindungsgemäß ist vorgesehen, dass ein erfindungsgemäßes Set mit einer zusätzlichen ersten Teileinrichtung ausgebildet ist, also als Set aus einer zweiten Teileinrichtung und mindestens zwei ersten Teileinrichtungen. Die Austrageinheit umfasst somit bereits alle Komponenten, die für die Verwendung an mehreren Patienten erforderlich sind. Dabei ist es von besonderem Vorteil, wenn die verschiedenen ersten Teileinrichtungen, die Teil eines solchen Sets sind, sich hinsichtlich der Geometrie der jeweiligen Nasenolive und/oder der Gestaltung der ersten Verwirbelungseinrichtungen unterscheiden, so dass die Austrageinheit an spezifische Bedürfnisse, insbesondere an die spezifischen Bedürfnisse von Erwachsenen einerseits und Kinder andererseits, anpassbar ist.

### KURZBESCHREIBUNG DER ZEICHNUNGEN

Weitere Vorteile und Aspekte der Erfindung ergeben sich außer aus den Ansprüchen auch aus der nachfolgenden Beschreibung von bevorzugten Ausführungsbeispielen der Erfindung, die anhand der Figuren erläutert sind. Dabei zeigen:
Fig. 1a und 1b eine erste Austrageinheit gemäß der vorliegenden Erfindung mit Adaptereinheit, wobei Fig. 1b eine vergrößerte Detailansicht darstellt, und
Fig. 2a und 2b eine zweite Austrageinheit gemäß der vorliegenden Erfindung mit einer Adaptereinheit, wobei Fig. 2b eine vergrößerte Detailansicht darstellt.

### DETAILLIERTE BESCHREIBUNG DER AUSFÜHRUNGSBEISPIELE

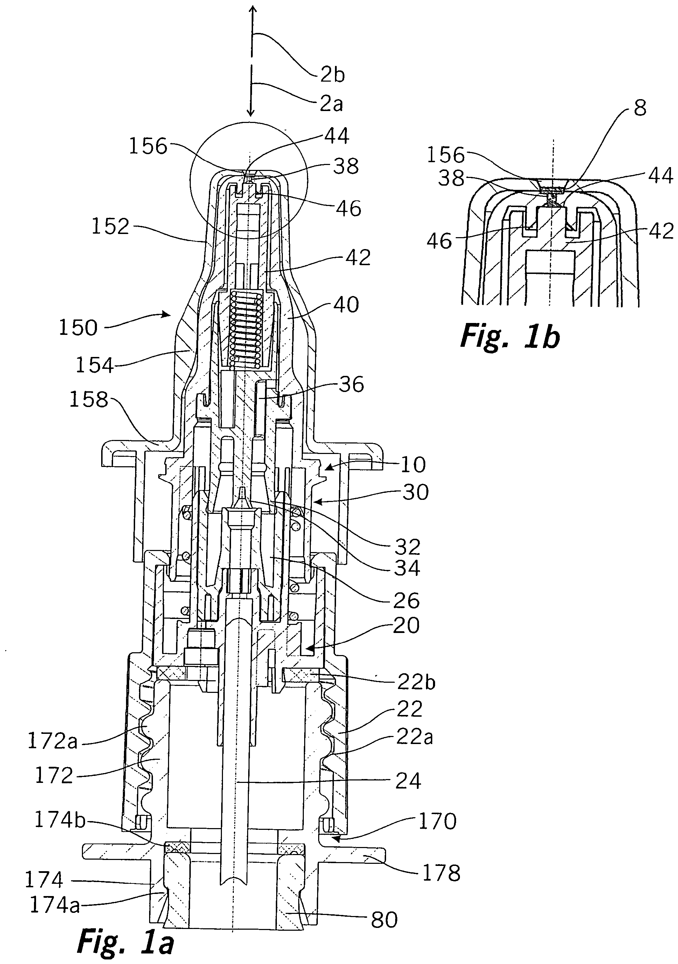

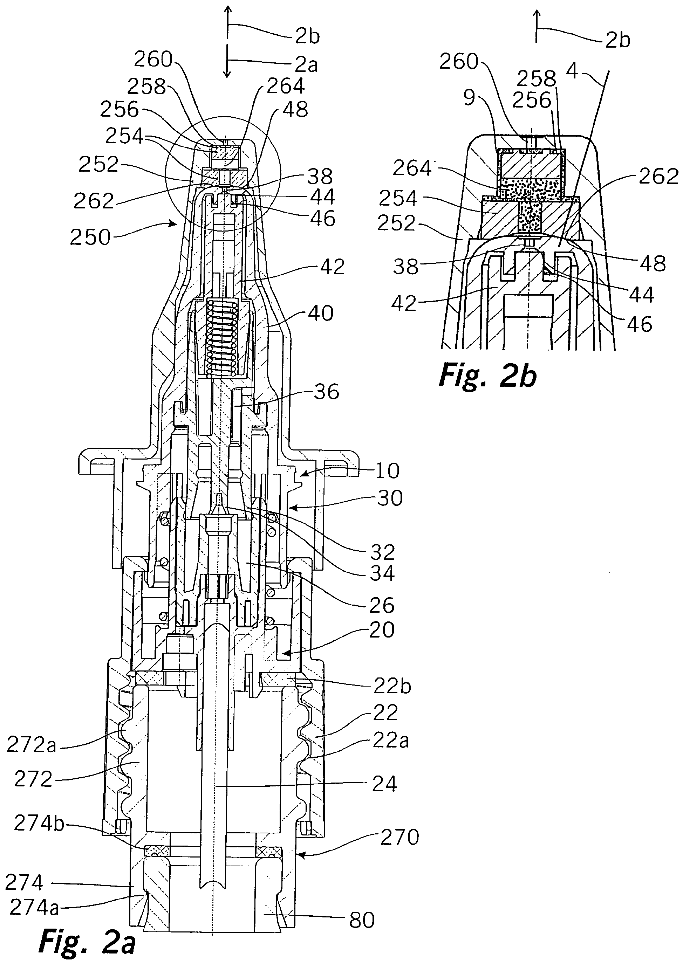

Die beiden Austrageinheiten gemäß der Fig. 1 und 2 weisen übereinstimmend jeweils drei Hauptkomponenten auf, die dafür ausgebildet sind, durch einen Endanwender, beispielsweise einen das mit der Austrageinheit austragbare Medium verabreichenden Arzt, zusammengesetzt zu werden. Bei diesen drei Baueinheiten handelt es sich um eine Grundeinheit 10, die bei beiden Ausführungsformen der Fig. 1 und 2 im Wesentlichen identisch ausgebildet ist und die die zweite Teileinrichtung im Sinne der vorliegenden Erfindung darstellt, um einen Aufsatz 150, 250 der die erste Teileinrichtung im Sinne der vorliegenden Erfindung darstellt, sowie um eine Adaptereinheit 170, 270.

Aufgrund der weitgehend übereinstimmenden Gestaltung der Grundeinheit 10 wird diese zunächst erläutert. Die Grundeinheit 10 weist zwei gegeneinander in einer Pumprichtung 2a translativ verlagerbare Baugruppen 20, 30 auf. Die untere Baugruppe 20 umfasst einen mit Innengewinde 22a und elastischem Dichtring 22b ausgebildeten Verbindungsanschluss 22. Durch diesen Verbindungsanschluss 22 ragt ein abschnittsweise als Steigrohr ausgebildeter Einlasskanal 24 hindurch, der in eine durch Abschnitte der unteren Baugruppe 20 gebildete Pumpkammer 26 mündet. Korrespondierend hierzu weist die obere Baugruppe 30 einen in die Pumpkammer 26 hineinragenden Kolben 32 auf, der durch Relativverlagerung der Baugruppen 20, 30 in der Lage ist, den Einlasskanal 24 mittels eines Verschlussabschnitts 34 zu verschließen, um im Zuge einer weiter gehenden Relativverlagerung der Baugruppen 20, 30 das Volumen der Pumpkammer 26 zu verringern und dadurch den Austragvorgang zu bewirken. In der oberen Baugruppe 30 ist zu diesem Zweck ein Teilabschnitt 36 eines Auslasskanals vorgesehen, der zu einer Auslassöffnung 38 führt, die an dem distalen Ende einer Nasenolive 40 angeordnet ist. Innerhalb der Nasenolive 40 ist ein Ventilschieber 42 vorgesehen, der federkraftbeaufschlagt gegen die Auslassöffnung 38 gepresst wird und diese erst bei Anliegen eines ausreichenden Mediendrucks im Auslasskanal 36 freigibt. Als Gegenabschnitt zum Ventilschieber 40 ist die Auslassöffnung 38 umgebend eine Dichtfläche 44 an der Innenseite der Nasenolive 40 vorgesehen. Dem Auslassventil 42, 44 unmittelbar vorgeschaltet ist eine Verwirbelungseinrichtung, die durch schräg gestellte Verwirbelungsflächen 46 gebildet wird, welche der bei geöffnetem Ventil 40, 42 von der Pumpkammer 26 zur Austragöffnung 38 strömenden Flüssigkeit einen Drall verleihen, der dazu führt, dass das Medium beim Hindurchtreten durch die Auslassöffnung 38 einen konusförmigen Sprühstrahl bildet.

Die beschriebene Grundeinheit 10 ist derart ausgebildet, dass sie ohne Verwendung der Adaptereinheiten 170, 270 unmittelbar auf einer hierfür vorgesehenen Medienflasche aufgeschraubt werden kann. Sie ist weiterhin dafür ausgebildet, ohne einen Aufsatz 150, 250 als nasale Austrageinheit Verwendung zu finden.

Um diese Grundeinheit 10 insbesondere auch als Austrageinheit für Massenimpfungen verwenden zu können, sind die Aufsätze 150, 250 vorgesehen.

Die Austrageinheit der Fig. 1a und 1b verdeutlicht ein erstes Konzept. Der Aufsatz 150 ist dafür ausgebildet, auf die Nasenolive 40 aufgesetzt zu werden. Während seine Außenform in einem oberen Bereich die Form einer Nasenolive 152 aufweist, sind an der Innenseite Rippen 154 vorgesehen, die nach Aufsetzen des Aufsatzes 150 auf die Nasenolive 40 eine sichere Fixierung bilden. Am distalen Ende weist der Aufsatz 150 eine Austragöffnung 156 auf, die größer als die Austragöffnung 38 der Grundeinheit 10 ausgebildet ist. Am unteren Ende des Aufsatzes 150 ist eine sich radial nach außen erstreckende Fingerauflage 158 vorgesehen.

Nachdem dieser Aufsatz 150 auf die Nasenolive 40 der Grundeinheit 10 aufgesetzt ist, kann durch eine Kraftbeaufschlagung der Fingerauflage 158 in Pumprichtung 2a nach unten ein Austragvorgang verursacht werden, da durch diese Kraftbeaufschlagung die obere Baugruppe 30 der Grundeinheit 10 gemeinsam mit dem Aufsatz 150 gegenüber der unteren Baugruppe 20 der Grundeinheit 10 verlagert wird, so dass das in der Pumpkammer 26 vorhandene Medium aus der Pumpkammer 26 herausgedrückt wird. Das Medium wird in den Auslasskanal 36 gefördert, wo der Flüssigkeitsdruck steigt, bis das Auslassventil 40, 42 öffnet und das durch die Verwirbelungsflächen 46 mit einem Drall versehene Medium in Form eines Sprühstrahls durch die Austragöffnungen 38, 156 ausgetragen wird.

Durch die Kraftbeaufschlagung der Fingerauflage 158 wird dabei nicht nur der genannte Austrag erzielt, sondern gleichzeitig gewährleistet, dass der Aufsatz 150 an die Nasenolive 40 der Grundeinheit 10 angepresst wird, so dass kein Medium zwischen dem Aufsatz 150 und der Außenseite der Nasenolive 40 verloren geht.

Nach erfolgtem Austragvorgang kann der Aufsatz 150 in einfacher Weise von der Grundeinheit 10 in entgegengesetzter Richtung 2b nach oben abgezogen werden, so dass hierdurch potentiell kontaminierte Flächen des Aufsatzes 150 entfernt werden. Bei der Gestaltung der Fig. 1a und 1b kann jedoch eine Kontamination im Bereich der Austragöffnung 38 der Grundeinheit 10 verbleiben. Es wird daher bevorzugt, dass vor dem Aufsetzen eines neuen Aufsatzes 150 zunächst die die Auslassöffnung 38 mit einer desinfizierenden Flüssigkeit desinfiziert wird. Dabei werden auch verbleibende Medienreste im Bereich der Austragöffnung 38 entfernt. Diese Medienreste stellen hinsichtlich ihres Volumens jedoch einen vernachlässigbaren Verlust dar.

Nach Aufsetzen eines neuen Aufsatzes 150 kann die Austrageinheit bei einem weiteren Patienten angewendet werden, ohne dass die Gefahr einer Querkontamination besteht, da alle möglicherweise kontaminierten Flächen entfernt bzw. desinfiziert wurden. Aus Fig. 1b ist durch die gepunktet dargestellten Flächen 8 ersichtlich, welch geringe Menge des Mediums bei einem Wechsel des Aufsatzes 150 und ggf. einer Desinfektion der Grundeinheit 10 verloren geht. Bei der Ausführungsform der Fig. 1a und 1b handelt es sich um weniger als 0,1 µl. Da das Medium im Auslasskanal 36 durch eine etwaige Kontamination nicht tangiert wäre und daher nicht entfernt werden muss, kann nach dem Wechsel des Aufsatzes 150 ohne erneutes Primen unmittelbar wieder Medium ausgetragen werden.

Der Aufsatz 250 der Gestaltung der Fig. 2a und 2b weist einen vom Aufsatz 150 abweichenden komplexeren Aufbau auf. Wie insbesondere an der Fig. 2b zu erkennen ist, verfügt dieser zweite Aufsatz über einen kappenförmiges Bauteil 252, in welches ein einstückiges Bauteil 254 aus einem weicheren Kunststoff eingepresst ist. Die obere Stirnfläche 256 des inneren Bauteils bildet zusammen mit der innenseitigen Fläche 258 des kappenförmigen Außenbauteils 252 eine Verwirbelungseinrichtung, die einer Austragöffnung 260 unmittelbar vorgeschaltet ist. Diese Verwirbelungseinrichtung weist wiederum in nicht näher dargestellter Weise schräg gestellte Verwirbelungsflächen auf, die dem vorbeiströmenden Medium einen Drall verleihen. Das Innenbauteil 254 weist auf der gegenüberliegenden Seite in Richtung der Nasenolive 40 der Grundeinheit 10 weisend eine Dichtfläche 262 auf, die im gekoppelten Zustand der Fig. 2a und 2b abdichtend an einem Dichtflächenabschnitt 48 der Grundeinheit 10 anliegt.

Wie bereits im Zusammenhang mit den Fig. 1a und 1b beschrieben wurde, ist auch die Grundeinheit 10 der Austrageinheit der Fig. 2a und 2b separat funktionstauglich. Wenn die Grundeinheit 10 jedoch mit dem Aufsatz 250 verwendet wird, bildet das Medium nach Durchströmen der Auslassöffnung 38 kein Sprühstrahl aus, sondern wird weiterhin als einheitlicher Flüssigkeitsstrom entlang des Aufsatzkanals 264 bis zur Verwirbelungseinrichtung 256, 258 weitergeleitet. Dort erhält der Flüssigkeitsstrom durch die Verwirbelungseinrichtung 256, 258 erneut einen Drall, der zu einem Austrag des Mediums durch die Austragöffnung 260 hindurch in Form eines konischen Sprühstrahls an die Umgebung erfolgt.

Da die Verwirbelungseinrichtung 46 bei einer Verwendung der Grundeinheit 10 mit dem Aufsatz 250 nicht benötigt wird, kann sie auch entfallen. In dem Fall ist eine Verwendung der Grundeinheit 10 nur noch gemeinsam mit dem Aufsatz 250 möglich.

Eine Kontamination durch einen Patienten, der die Austrageinheit der Fig. 2a und 2b verwendet, findet gegebenenfalls vor allem an der Außenoberfläche des kappenförmigen Außenbauteils 252 statt. Es hat sich gezeigt, dass sich eine Kontamination nur in sehr seltenen Fällen bis in die Verwirbelungseinrichtung 256, 258 fortsetzt. Selbst wenn dies geschieht, ist diese Kontamination von der Grundeinheit 10 zusätzlich durch die Flüssigkeit im Aufsatzkanal 264 getrennt, so dass eine Kontamination keinesfalls bis an die Außenseite der Nasenolive 40 der Grundeinheit 10 gelangt.

Wie auch bei der ersten Ausführungsform der Fig. 1a und 1b wird nach der Verwendung der Grundeinheit 10 mit Aufsatz 250 zur Verabreichung eines Impfstoffs an einen ersten Patienten der Aufsatz 250 für einen zweiten Patienten ausgetauscht. Dabei geht lediglich das in Fig. 2b gepunktet dargestellte Innenvolumen 9 von Medium verloren. Es handelt sich dabei allerdings nur um 3 bis 5 µl. Auf eine Desinfektion der Nasenolive 40 der Grundeinheit 10 kann üblicherweise verzichtet werden, da eine eventuelle Kontamination sich nicht bis zur Nasenolive 40 fortpflanzt. Dies wird auch dadurch erreicht, dass die Dichtflächen 262, 48 einen Normalenvektor 4 aufweisen, der mit der Entkopplungsrichtung 2b einen Winkel von weniger als 30° einschließt, so dass sich beim Entkoppeln des Aufsatzes 250 von der Grundeinheit 10 zwischen diesen kein Unterdruck bildet, durch den kontaminiertes Medium bis an die Nasenolive 40 angesogen werden könnte. Stattdessen kommt es beim Entkoppeln unmittelbar zu einem Verlust des Berührkontaktes zwischen den Dichtflächen 48, 262.

Beide Austragvorrichtungen der Fig. 1 und 2 weisen jeweils eine Adaptereinheit 170, 270 auf. Diese verfügen an ihren oberen Enden über einen zweiten Verbindungsanschluss 172, 272, der mit einem Außengewinde 172a, 272a versehen ist. Dieses Außengewinde 172a, 272a ist an das Innengewinde 22a des ersten Verbindungsanschlusses 22 der Grundeinheit 10 angepasst. Am gegenüberliegenden unteren Ende verfügen die Adaptereinheiten 170, 270 jeweils über einen dritten Verbindungsanschluss 174, 274, der zylindrisch ausgebildet ist und an dessen Zylinderinnenseite jeweils eine Schnappkante 174a, 274a vorgesehen ist, die dem Zweck dient, auf einen Medienspeicher 80 aufgesetzt zu werden, der seinerseits nicht über einen Gewindeanschluss, sondern über einen zum dritten Verbindungsanschluss 170, 270 korrespondierenden Schnappverschluss führen. Zum Zwecke einer vollständigen Abdichtung ist der dritte Verbindungsanschluss 174, 274 jeweils mit einem umlaufenden elastischen Dichtelement 174b, 274b ausgestattet.

Die Adaptereinheiten 170, 270, die zusammen mit der Grundeinheit 10 ausgeliefert werden, erlauben es, die ursprünglich nicht zur Verbindung mit einem Medienspeicher des Typs des Medienspeichers 80 ausgebildete Grundeinheit 10 auf dem Umweg über die Adaptereinheiten 170, 270 an einen solchen Medienspeicher 80 anzuschließen. Hierdurch werden schnellere Reaktionszeiten bei der Versorgung des Marktes mit einer Vielzahl von Austrageinheiten für Medienspeicher des Typs des Medienspeichers 80 möglich, da die Grundeinheit 10 und die Aufsätze 150, 250 bereits in großen Stückzahlen vorgefertigt sein können und für den Fall, dass es sich herausstellt, dass Anschluss an einen Medienspeicher des Typs des Medienspeichers 80 erforderlich ist, um einfach und schnell produzierbare Adaptereinheiten 170, 270 ergänzt werden können.

Die Adaptereinheiten 170, 270 unterscheiden sich voneinander nur dadurch, dass die Adaptereinheit 170 der Austrageinheit der Fig. la in Form einer außenseitig angeformten Radialfläche einer Montagehilfe 178 aufweist, die das Aufschnappen des dritten Verbindungsanschlusses 170 auf den Medienspeicher 80 und das Koppeln des zweiten Verbindungsanschlusses 172 mit dem ersten Verbindungsanschluss 22 erleichtert.

## Patentansprüche

1. Set aus mindestens zwei ersten Teileinrichtungen (150, 250) und einer zweiten Teileinrichtung (10) zur Bildung einer Austrageinheit (10, 150, 250) zum Austrag pharmazeutischer Medien aus einem Medienspeicher (80) mit
- einer ersten Austragöffnung (156, 260),
- einer Pumpe (26, 32),
- einem Einlasskanal (24) zur Verbindung des Medienspeichers (80) mit der Pumpe (26, 32) und
- einem Auslasskanal (36, 264) zur Verbindung der Pumpe (26, 32) mit der ersten Austragöffnung (156, 260),
wobei
- jeweils eine erste Austragöffnung (156, 260) Teil jeweils der ersten Teileinrichtungen (150, 250) der Austrageinheit ist,
- die Pumpe (26,32), zumindest ein Teilabschnitt (36) des Auslasskanals (36) sowie ein im Auslasskanal (36) vorgesehenes Auslassventil (42, 44) Teil der zweiten Teileinrichtung (10) der Austrageinheit sind, und
- die ersten Teileinrichtungen (150, 250) als nacheinander werkzeuglos an die zweite Teileinrichtung (10) ankoppelbare und von der zweiten Teileinrichtung (10) entkoppelbare Teileinrichtung (150, 250) ausgebildet sind und
- ein Innenvolumen (8, 9) eines Teilabschnitts des Auslasskanals, der sich vom Auslassventil (42, 44) zur ersten Austragöffnung (156, 260) erstreckt kleiner als 15 µl ist, vorzugsweise kleiner als 5 µl, insbesondere vorzugsweise kleiner als 1 µl.

2. Set aus mindestens zwei ersten Teileinrichtungen (250) und einer zweiten Teileinrichtung (10) zur Bildung einer Austrageinheit (250) zum Austrag pharmazeutischer Medien aus einem Medienspeicher (80) mit
- einer ersten Austragöffnung (260),
- einer Pumpe (26, 32),
- einem Einlasskanal (24) zur Verbindung des Medienspeichers (80) mit der Pumpe (26, 32) und
- einem Auslasskanal (264) zur Verbindung der Pumpe (26, 32) mit der ersten Austragöffnung (260),
wobei
- die erste Austragöffnung (260), zumindest ein Teilabschnitt (264) des Auslasskanals (36, 264) sowie eine im Auslasskanal vorgesehene erste Verwirbelungseinrichtung (256, 258) Teil jeweils der ersten Teileinrichtungen (250) sind und
- die Pumpe (26,32) und zumindest ein Teilabschnitt (36) des Auslasskanals (36, 264) Teil der zweiten Teileinrichtung (10) sind,
- die erste Teileinrichtung (250) als werkzeuglos an die zweite Teileinrichtung (10) ankoppelbare und von der zweiten Teileinrichtung (10) entkoppelbare Teileinrichtung (250) ausgebildet ist und
- ein Innenvolumen (9) des in der ersten Teileinrichtung (250) vorgesehenen Teilabschnitts (264) des Auslasskanals (36, 264) kleiner als 15 µl ist, vorzugsweise kleiner als 5 µl, insbesondere vorzugsweise kleiner als 1 µl.

3. Set nach einem der vorstehenden Ansprüche,
**dadurch gekennzeichnet, dass**
die ersten Teileinrichtungen (150, 250) jeweils einen Applikator, vorzugsweise in Form einer Nasenolive (152, 252), aufweisen, an dem die erste Austragöffnung (156, 260) vorgesehen ist.

4. Set nach einem der vorstehenden Ansprüche,
**dadurch gekennzeichnet, dass**
- die zweite Teileinrichtung (10) einen Applikator (40), insbesondere in Form einer Nasenolive (40), mit einer zweiten Austragöffnung (38) aufweist, und
- die ersten Teileinrichtungen (150, 250) zum Aufsetzen auf diesen Applikator (40) der zweiten Teileinrichtung (10) ausgebildet sind.

5. Set nach Anspruch 4,
**dadurch gekennzeichnet, dass**
die zweite Teileinrichtung (10) eine der zweiten Austragöffnung (38) vorgeschaltete zweite Verwirbelungseinrichtung (46) aufweist.

6. Set nach einem der Ansprüche 4 und 5,
**dadurch gekennzeichnet, dass**
- die ersten Teileinrichtungen (150,250) und die zweite Teileinrichtung (10) jeweils eine Nasenolive (40, 152, 252) aufweisen und die Nasenoliven (152, 252) der ersten Teileinrichtungen eine von der Nasenolive (40) der zweiten Teileinrichtung abweichende Geometrie aufweisen, und/oder
- die ersten Teileinrichtungen (150, 250) und die zweite Teileinrichtung (10) jeweils eine Verwirbelungseinrichtung (46, 256, 258) aufweisen und die Verwirbelungseinrichtungen (256, 258) der ersten Teileinrichtungen (250) zur Erzeugung eines Sprühverhaltens ausgebildet ist, das sich vom Sprühverhalten der Verwirbelungseinrichtung (46) der zweiten Teileinrichtung (10) unterscheidet.

7. Set nach einem der vorstehenden Ansprüche,
**dadurch gekennzeichnet, dass**
die ersten Teileinrichtungen (250) jeweils einen kappenförmigen ersten Außenkörper (252) und einen darin eingesetzten Innenkörper (254) aufweisen, wobei der Innenkörper (254) jeweils
- zumindest abschnittsweise die erste Verwirbelungseinrichtung (256, 258) bildet und/oder
- eine Dichtfläche (262) zur Verfügung stellt, die im gekoppelten Zustand an einer äußeren Oberfläche (48) der zweiten Teileinrichtung (10) anliegt.

8. Set nach einem der vorstehenden Ansprüche,
**dadurch gekennzeichnet, dass**
an den ersten Teileinrichtungen (250) und an der zweiten Teileinrichtung (10, 250) jeweils eine Dichtfläche (48, 262) vorgesehen ist, die im gekoppelten Zustand einer der ersten und der zweiten Teileinrichtung (10, 250) aneinander anliegen und dadurch den Auslasskanal (36, 264) nach außen umgebend gemeinsam abdichten, wobei ein Normalenvektor (4) auf diesen Dichtflächen (48, 262) mit einer Entkopplungsrichtung (2b) der Teileinrichtungen (10, 250) einen Winkel kleiner 60° einschließt, vorzugsweise kleiner 30°, insbesondere kleiner 15°.

9. Set nach einem der vorstehenden Ansprüche,
**dadurch gekennzeichnet, dass**
an den ersten Teileinrichtungen (150, 250) jeweils eine Fingerauflage (158) vorgesehen ist.

10. Set nach einem der vorstehenden Ansprüchen,
**dadurch gekennzeichnet, dass**
die ersten Teileinrichtungen (150; 250) und die zweite Teileinrichtung (10) für eine rein kraftschlüssige Kopplung ausgebildet sind, insbesondere für eine hinterschneidungsfreie Kopplung.

11. Set nach einem der vorstehenden Ansprüche,
**dadurch gekennzeichnet, dass**
die ersten Teileinrichtungen (150, 250) in Hinblick auf
- die Geometrie der jeweiligen Nasenolive (152, 252) und/oder
- die Gestaltung der ersten Verwirbelungseinrichtungen (256,258) unterschiedlich ausgebildet sind.

## Claims

1. Set comprising at least two first part devices (150, 250) and a second part device (10) for the formation of a discharge unit (10, 150, 250) for the discharge of pharmaceutical media from a medium store (80) having
- a first discharge opening (156, 260),
- a pump (26, 32),
- an inlet duct (24) for the connection of the medium store (80) to the pump (26, 32), and
- an outlet duct (36, 264) for the connection of the pump (26, 32) to the first discharge opening (156, 260),
- in each case a first discharge opening (156, 260) being a part in each case of the first part devices (150, 250) of the discharge unit,
- the pump (26, 32), at least one part section (36) of the outlet duct (36) and an outlet valve (42, 44) which is provided in the outlet duct (36) being part of the second part device (10) of the discharge unit, and
- the first part devices (150, 250) being configured as part devices (150, 250) which can be coupled one after another to the second part device (10) without tools and can be decoupled from the second part device (10), and
- an internal volume (8, 9) of a part section of the outlet duct which extends from the outlet valve (42, 44) to the first discharge opening (156, 260) being smaller than 15 µl, preferably smaller than 5 µl, particularly preferably smaller than 1 µl.

2. Set comprising at least two first part devices (250) and a second part device (10) for the formation of a discharge unit (250) for the discharge of pharmaceutical media from a medium store (80) having
- a first discharge opening (260),
- a pump (26, 32),
- an inlet duct (24) for the connection of the medium store (80) to the pump (26, 32), and
- an outlet duct (264) for the connection of the pump (26, 32) to the first discharge opening (260),
- the first discharge opening (260), at least one part section (264) of the outlet duct (36, 264) and a first swirl device (256, 258) which is provided in the outlet duct being part in each case of the first part devices (250), and
- the pump (26, 32) and at least one part section (36) of the outlet duct (36, 264) being part of the second part device (10),
- the first part device (250) being configured as a part device (250) which can be coupled to the second part device (10) without tools and can be decoupled from the second part device (10), and
- an internal volume (9) of the part section (264) of the outlet duct (36, 264) which is provided in the first part device (250) being smaller than 15 µl, preferably smaller than 5 µl, particularly preferably smaller than 1 µl.

3. Set according to either of the preceding claims,
**characterized in that**
the first part devices (150, 250) in each case have an applicator, preferably in the form of a nasal olive (152, 252), on which the first discharge opening (156, 260) is provided.

4. Set according to one of the preceding claims,
**characterized in that**
- the second part device (10) has an applicator (40), in particular in the form of a nasal olive (40), with a second discharge opening (38), and
- the first part devices (150, 250) are configured to be placed onto the said applicator (40) of the second part device (10).

5. Set according to Claim 4,
**characterized in that**
the second part device (10) has a second swirl device (46) which is connected upstream of the second discharge opening (38).

6. Set according to either of Claims 4 and 5, **characterized in that**
- the first part devices (150, 250) and the second part device (10) in each case have a nasal olive (40, 152, 252), and the nasal olives (152, 252) of the first part devices have a geometry which differs from the nasal olive (40) of the second part device, and/or
- the first part devices (150, 250) and the second part device (10) in each case have a swirl device (46, 256, 258), and the swirl devices (256, 258) of the first part devices (250) are configured to generate a spray behaviour which differs from the spray behaviour of the swirl device (46) of the second part device (10).

7. Set according to one of the preceding claims,
**characterized in that**
the first part devices (250) in each case have a cap-shaped first outer body (252) and an inner body (254) which is inserted into the latter, the inner body (254) in each case
- forming the first swirl device (256, 258) at least in sections, and/or
- providing a sealing face (262) which bears against an outer surface (48) of the second part device (10) in the coupled state.

8. Set according to one of the preceding claims,
**characterized in that**
a sealing face (48, 262) is provided in each case on the first part devices (250) and on the second part device (10, 250), which sealing faces (48, 262) bear against one another in the coupled state of one of the first and the second part device (10, 250) and, as a result, together seal the outlet duct (36, 264) in a surrounding manner to the outside, a normal vector (4) of the said sealing faces (48, 262) enclosing an angle of smaller than 60°, preferably of smaller than 30°, in particular of smaller than 15° with a decoupling direction (2b) of the part devices (10, 250).

9. Set according to one of the preceding claims,
**characterized in that**
a finger rest (158) is provided in each case on the first part devices (150, 250).

10. Set according to one of the preceding claims,
**characterized in that**
the first part devices (150; 250) and the second part device (10) are configured for purely non-positive coupling, in particular for undercut-free coupling.

11. Set according to one of the preceding claims,
**characterized in that**
the first part devices (150, 250) are of different configuration with regard to
- the geometry of the respective nasal olive (152, 252) and/or
- the design of the first swirl devices (256, 258).

## Revendications

1. Ensemble d'au moins deux premiers dispositifs partiels (150, 250) et d'un deuxième dispositif partiel (10) pour la formation d'une unité de distribution (10, 150, 250) pour la distribution d'agents pharmaceutiques à partir d'un réservoir d'agent (80), avec
- un premier orifice de distribution (156, 260),
- une pompe (26, 32),
- un canal d'admission (24) pour le raccordement du réservoir d'agent (80) à la pompe (26, 32), et
- un canal d'évacuation (36, 264) pour le raccordement de la pompe (26, 32) au premier orifice de distribution (156, 260),
dans lequel
- chaque fois un premier orifice de distribution (156, 260) fait respectivement partie des premiers dispositifs partiels (150, 250) de l'unité de distribution,
- la pompe (26, 32), au moins une partie partielle (36) du canal d'évacuation (36) ainsi qu'une soupape d'évacuation (42, 44) prévue dans le canal d'évacuation (36) font partie du deuxième dispositif partiel (10) de l'unité de distribution, et
- les premiers dispositifs partiels (150, 250) sont réalisés sous la forme d'un dispositif partiel (150, 250) pouvant, l'un après l'autre et sans outil, être couplé au deuxième dispositif partiel (10) et découplé du deuxième dispositif partiel (10), et
- un volume intérieur (8, 9) d'une partie partielle du canal d'évacuation, qui s'étend de la soupape d'évacuation (42, 44) au premier orifice de distribution (156, 260), est inférieur à 15 µl, de préférence inférieur à 5 µl, en particulier de préférence inférieur à 1 µl.

2. Ensemble d'au moins deux premiers dispositifs partiels (250) et d'un deuxième dispositif partiel (10) pour la formation d'une unité de distribution (250) pour la distribution d'agents pharmaceutiques à partir d'un réservoir d'agent (80), avec
- un premier orifice de distribution (260),
- une pompe (26, 32),
- un canal d'admission (24) pour le raccordement du réservoir d'agent (80) à la pompe (26, 32), et
- un canal d'évacuation (264) pour le raccordement de la pompe (26, 32) au premier orifice de distribution (260), dans lequel
- le premier orifice de distribution (260), au moins une partie partielle (264) du canal d'évacuation (36, 264) ainsi qu'un premier dispositif de tourbillonnement (256, 258) prévu dans le canal d'évacuation font partie respectivement des premiers dispositifs partiels (250), et
- la pompe (26, 32) et au moins une partie partielle (36) du canal d'évacuation (36, 264) font partie du deuxième dispositif partiel (10),
- le premier dispositif partiel (250) est réalisé sous la forme d'un dispositif partiel (250) pouvant, sans outil, être couplé au deuxième dispositif partiel (10) et découplé du deuxième dispositif partiel (10), et
- un volume intérieur (9) de la partie partielle (264) du canal d'évacuation (36, 264) prévue dans le premier dispositif partiel (250), est inférieur à 15 µl, de préférence inférieur à 5 µl, en particulier de préférence inférieur à 1 µl.

3. Ensemble selon l'une quelconque des revendications précédentes, **caractérisé en ce que** les premiers dispositifs partiels (150, 250) présentent respectivement un applicateur, de préférence sous la forme d'une olive nasale (152, 252), sur lequel le premier orifice de distribution (156, 260) est prévu.

4. Ensemble selon l'une quelconque des revendications précédentes, **caractérisé en ce que**
- le deuxième dispositif partiel (10) présente un applicateur (40), en particulier sous la forme d'une olive nasale (40), avec un second orifice de distribution (38), et
- les premiers dispositifs partiels (150, 250) sont réalisés pour se placer sur cet applicateur (40) du deuxième dispositif partiel (10).

5. Ensemble selon la revendication 4, **caractérisé en ce que** le deuxième dispositif partiel (10) présente un second dispositif de tourbillonnement (46) installé avant le second orifice de distribution (38).

6. Ensemble selon une des revendications 4 et 5, **caractérisé en ce que**
- les premiers dispositifs partiels (150, 250) et le deuxième dispositif partiel (10) présentent respectivement une olive nasale (40, 152, 252) et les olives nasales (152, 252) des premiers dispositifs partiels présentent une géométrie différente de l'olive nasale (40) du deuxième dispositif partiel, et/ou
- les premiers dispositifs partiels (150, 250) et le deuxième dispositif partiel (10) présentent respectivement un dispositif de tourbillonnement (46, 256, 258) et les dispositifs de tourbillonnement (256, 258) des premiers dispositifs partiels (250) sont conçus pour produire un comportement de pulvérisation, qui se distingue du comportement de pulvérisation du dispositif de tourbillonnement (46) du deuxième dispositif partiel (10) .

7. Ensemble selon l'une quelconque des revendications précédentes, **caractérisé en ce que** les premiers dispositifs partiels (250) présentent respectivement un premier corps extérieur en forme de capuchon (252) et un corps intérieur (254) introduit dans celui-ci, dans lequel le corps intérieur (254)
- forme respectivement au moins partiellement le premier dispositif de tourbillonnement (256, 258) et/ou
- procure une face d'étanchéité (262), qui dans l'état couplé s'applique sur une surface extérieure (48) du deuxième dispositif partiel (10).

8. Ensemble selon l'une quelconque des revendications précédentes, **caractérisé en ce qu'**il est prévu sur les premiers dispositifs partiels (250) et sur le deuxième dispositif partiel (10, 250) respectivement une face d'étanchéité (48, 262), qui dans l'état couplé d'un des premiers et du deuxième dispositifs partiels (10, 250) s'appliquent l'une sur l'autre et rendent ainsi ensemble le canal d'évacuation (36, 264) étanche en l'entourant vers l'extérieur, dans lequel un vecteur normal (4) sur ces faces d'étanchéité (48, 262) forme avec une direction de découplage (2b) des dispositifs partiels (10, 250) un angle inférieur à 60°, de préférence inférieur à 30°, en particulier inférieur à 15°.

9. Ensemble selon l'une quelconque des revendications précédentes, **caractérisé en ce qu'**il est prévu sur les premiers dispositifs partiels (150, 250) chaque fois une surface d'appui de doigt (158).

10. Ensemble selon l'une quelconque des revendications précédentes, **caractérisé en ce que** les premiers dispositifs partiels (150, 250) et le deuxième dispositif partiel (10) sont conçus pour un couplage purement par adhérence, en particulier pour un couplage sans contre-dépouille.

11. Ensemble selon l'une quelconque des revendications précédentes, **caractérisé en ce que** les premiers dispositifs partiels (150, 250) sont réalisés sous forme différente en ce qui concerne
- la géométrie de l'olive nasale respective (152, 252) et/ou
- la forme des premiers dispositifs de tourbillonnement (256, 258).
